# EUROPEAN PATENT APPLICATION

(11) **EP 2 752 482 A1**
(43) Date of publication of application: **09.07.2014**
(21) Application number: 11871577.0
(22) Date of filing: 29.08.2011
(51) Int. Cl.: C12M 3/00

(54) **CULTURING SHEET, CULTURING EQUIPMENT MATERIAL, AND MANUFACTURING METHOD**

(71) Applicant: Hitachi, Ltd., Tokyo 100-8280 (JP)
(72) Inventor: YAMAMOTO Jiro, Tokyo 100-8280 (JP); ITABASHI, Naoshi, Tokyo 100-8280 (JP); SAITO, Taku, Tokyo 100-8280 (JP); HISADA, Akiko, Tokyo 100-8280 (JP); SONODA, Hiroshi, Tokyo 100-8280 (JP)
(74) Representative: MERH-IP Matias Erny Reichl Hoffmann
(86) International application number: PCT/JP2011/069504
(87) International publication number: WO 2013/030940

(57) **Abstract**

An operating efficiency of an observer is considerably restricted since it is not known at which position a culturing cell is disposed among a great number of pieces of holes of a culturing sheet a region of which is separated into the holes by partitioning. The culturing sheet is configured by a partitioning wall 102, a hole 101 isolated by the partitioning wall 102, a local culturing region 103 formed with plural local culturing region pillars 105 a height of which is lower than that of the partitioning wall 102 at a portion of a bottom face, and identification mark pillars 106 formed at an identification mark region 104 which differs from the culturing region at the bottom face of the hole. An identification mark is prevented from being unable to be optically recognized by adhering a spheroid to the identification mark region 104 by making a diameter and a height of the identification mark pillar smaller than a diameter and a height of the local culturing pillar 105.

## Description

### Technical Field

The present invention relates to a technology of culturing an animal or a vegetable cell by using a culturing equipment material, and forming a granular tissue (three-dimensional tissue) and a lamina tissue (two-dimensional plane tissue) of the cell.

### Background Art

An in vitro assay utilizing cells is requested by substituting for an animal experiment in a process of developing medical supplies. Particularly, an activity of applying the assay to screening of a drug candidate substance, and a toxicity/metabolism test has been active.

Although an approach to a substitution method utilizing a cell by substituting for a conventional animal experiment has vigorously been tried in such a background, there is frequently a limit in predicting a clinical reaction. This is because it is conceived that a cell is not constructed by a structure simulating a structure of an actual living organism according to the culturing methods (Nonpatent Literature 1). Hence, formation of a three-dimensional tissue achieving a function nearer to that of a mode of life have been tried, and formation of a three-dimensional tissue has been succeeded in various cell species.

There has been developed a sheet (nanopillar sheet) for culturing formed on a sheet surface regularly aligned with extremely fine uniform protrusions as a material for forming a three-dimensional structure of a cell. However, there poses a problem that the formed three-dimensional tissue is high in a property of being ablated from the material (Patent Literature 1) and is lost in a procedure of exchanging culture media. There also poses a problem that a grain size of the formed three-dimensional tissue cannot be controlled, and therefore, a size thereof is not uniform, and performances of the respective three-dimensional tissues are dispersed, which is premature as a practical forming method.

Hence, a method shown in Patent Literature 2 has been conceived in order to form a three-dimensional tissue having uniform grain sizes.

### Citation List

### Patent Literatures

Patent Literature 1: Japanese Unexamined Patent Application Publication No. 2005-312343
Patent Literature 2: WO2010/150521
Patent Literature 3: Japanese Unexamined Patent Application Publication (Translation of PCT Application) No. 2000-504299

### Nonpatent Literature

Nonpatent Literature 1: "The Use of 3-D Cultures for High-Throughput Screening: The Multicellular Spheroid Model" Leoni A. Kunz-Schughartm James P. Freyer, Ferdinand Hofsteadter, and Reinhard Ebner J Biomol Screen, 9: 273-285(2004)

### Summary of Invention

### Technical Problem

An explanation will be given of a cell culturing equipment material of a multiwells plate type pasted with a culturing sheet in relation to a culturing equipment material of Patent Literature 2 described above in reference to Fig. 2. As shown in Fig. 2, a frame referred to as a well 111 is formed at a culturing vessel 110 which becomes a culturing equipment material of a multiwells plate type. A culturing sheet 100 is pasted to a bottom face of the frame. A surface of the culturing sheet 100 to which a cell adheres is formed with holes 101 including a local culturing region 103 installed with a pillar 105 configuring a protrusion, that is, a three-dimensional recesses and protrusions structure, and respectively isolated from each other by partitioning walls 102 higher than the protrusions.

According to a diameter, a pitch, and a height of the protrusion, values thereof optimum for forming a three-dimensional aggregate referred to as spheroid are determined depending on cell species. Therefore, a cell can be fixed only to the local culturing region 103 by configuring the optimum values. Disseminated cells can be limited to only within the holes by partitioning the culturing regions by the holes. Therefore, the cells are aggregated by moving the cells while a number of the cells are restricted to some degree by which a single spheroid is formed. It is expected by restricting the number of the cells that the spheroid is uniform in size and is homogeneous, which is effective for cell assay. The culturing equipment material is manufactured by summarizingly transcribing a die of a prescribed shape formed on a silicon substrate onto a resin such as polystyrene.

A method of evaluating the cell cultured in this way is carried out by, for example, observing a reaction of the cell before and after adding a drug by using an optical microscope. A cell is ordinarily cultured for over several days or several weeks, and the form of the cell changes every moment. As a result, in a case where a pitch of a finely partitioned culturing region, that is, a hole is made to be, for example, 220 µm, and a diameter of the well is made to be 8 mm, there are about 8000 pieces of the holes in a single well, and it is difficult to instantaneously find out a particular location. In a case where there are 8000 pieces of the partitioned culturing regions as described above, even when a record thereof remains by photograph or the like, in a case of seeing the record at later time, it is difficult to specify at which location the particular location is disposed. Incidentally, although a number of pieces of the holes per one well is changed by a size of the well and a size of the hole, only a degree of a difficulty of finding out the particular location differs, and the difficulty essentially remains unchanged.

In a case of industrially manufacturing a culturing sheet, it is necessary to inspect a defect of the culturing sheet or a die. When the defect is inspected, a defect portion and its location need to be specified. In that case, defect coordinates are calculated from coordinates relative to a specific hole as a reference, or absolute coordinates of a device after adjusting straight moving performance of an inspected object and a measuring device. However, once the culturing sheet is removed from an inspection device, coordinates information configuring the reference is lost, and a location of the defect is not known. In consideration of the fact that it is necessary that a person finally confirms the defect by an image, and determines whether the defect is to be modified, or whether the defect is abandoned as an unmodifiable killer defect, it is necessary to devise to specify a defect portion easily not as simple coordinates but as an image.

There is a case where cells cultured on a cell culturing sheet by adding or without adding a drug are collected to separate vessels, and evaluated by an analyzing device in order to observe a reaction by the drug. In the cultured cells, inactive dead cells are developed partially. In that case, the development becomes a noise in drug screening, and sensitivity is lowered thereby. Therefore, cells which become the noise are to be removed previously. Also in this case, it is indispensable to specify locations thereof. An active cell absorbs oxygen and discharges carbon dioxide, and therefore, life/death of the cell can be determined by measuring a concentration of carbon dioxide of each hole as an example of a method of measuring an activity of the cell.

On the other hand, there is a description in Patent Literature 3 that an identification mark is transcribed onto a bottom portion of a microwell as a method of specifying coordinates thereof. However, in a case of simply marking a plate, a cell senses a structure of the mark portion and is adsorbed to the mark portion, and the mark cannot be read by being shielded by the cell. Or, heights of a hole bottom portion to which a cell is dropped and an identification mark portion significantly differ from each other, and therefore, a cell shape and a mark cannot be observed by the same focal length and read simultaneously.

It is an object of the present invention to provide a culturing sheet, a culturing equipment material, and a manufacturing method thereof by which a three-dimensional tissue and an identification mark can simultaneously be read by resolving the problem described above.

### Solution to Problem

In order to achieve the object described above, according to the present invention, there are provided a culturing sheet for culturing a cell which is a culturing sheet including a partitioning wall, a hole isolated by the partitioning wall, a culturing region formed with plural protrusions a height of which is lower than a height of the partitioning wall at a portion of a bottom face of the hole, and an identification mark formed at a position different from a position of the culturing region at the bottom face of the hole, and a cultivating equipment material utilizing the same.

Also, in order to achieve the object described above, according to the present invention, there is provided a manufacturing method of a culturing sheet for culturing a cell which is a manufacturing method of a culturing sheet including a step of forming a die substrate of a culturing sheet including a partitioning wall, a hole isolated by the partitioning wall, a culturing region formed with plural protrusions a height of which is lower than a height of the partitioning wall at a portion of a bottom face of the hole, and an identification mark region formed at a position different from a position of the culturing region at the bottom face of the hole and formed with plural protrusions a height of which is lower than the height of the protrusion of the culturing region, and a step of pressing a material of the culturing sheet to the die substrate while heating the material. Advantageous Effects of Invention

Formation of a three-dimensional tissue of a cultured cell can be realized, and an identification mark can be read without being shielded by the three-dimensional tissue, and therefore, the three-dimensional tissue and a two-dimensional plane tissue can be evaluated and managed by applying the present invention. Brief Description of Drawings

Fig. 1 is a view showing a culturing sheet and a hole structure in the culturing sheet according to a first embodiment.

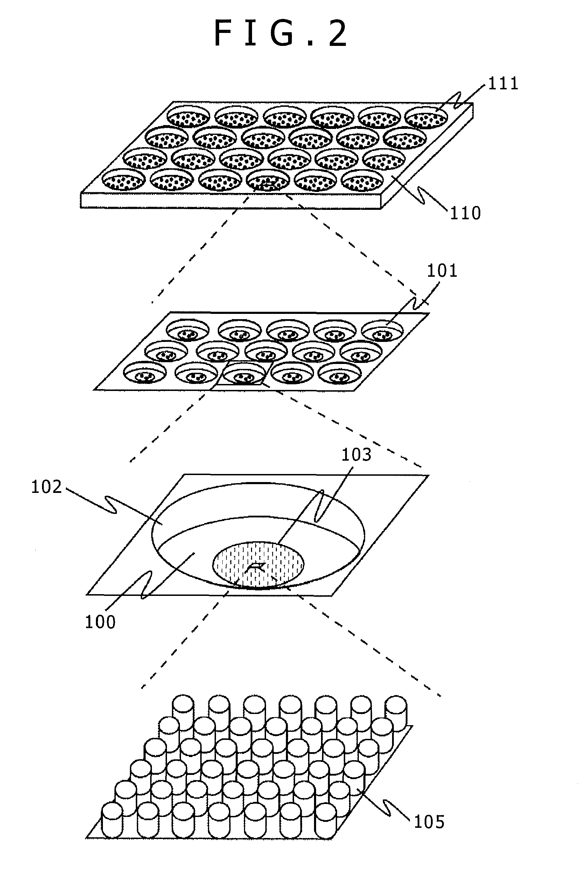

Fig. 2 is a view showing a culturing equipment material, a culturing sheet, and a hole structure in the culturing sheet of a background art.

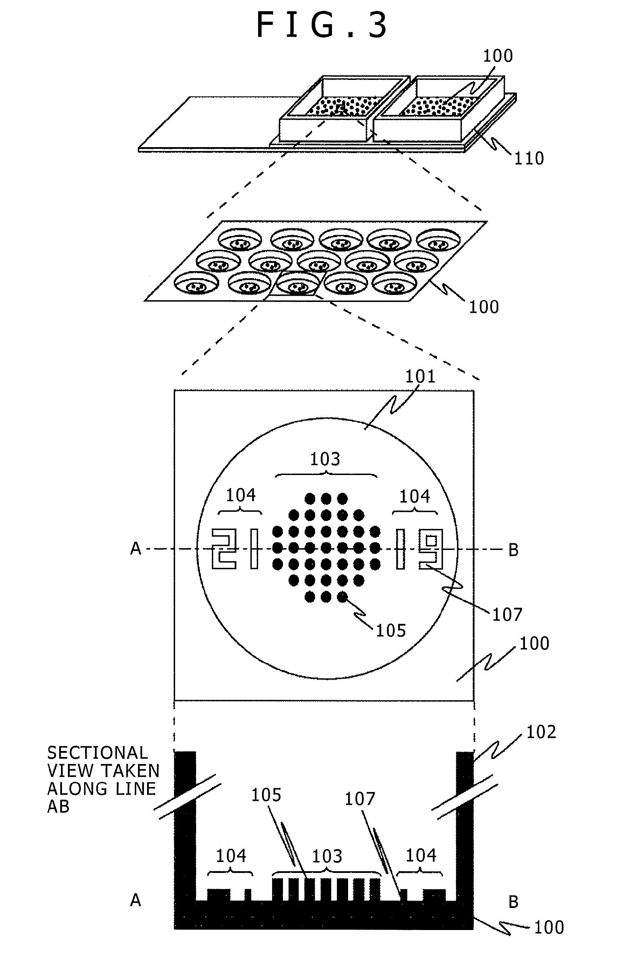

Fig. 3 is a view showing a culturing sheet and a hole structure in the culturing sheet according to a second embodiment.

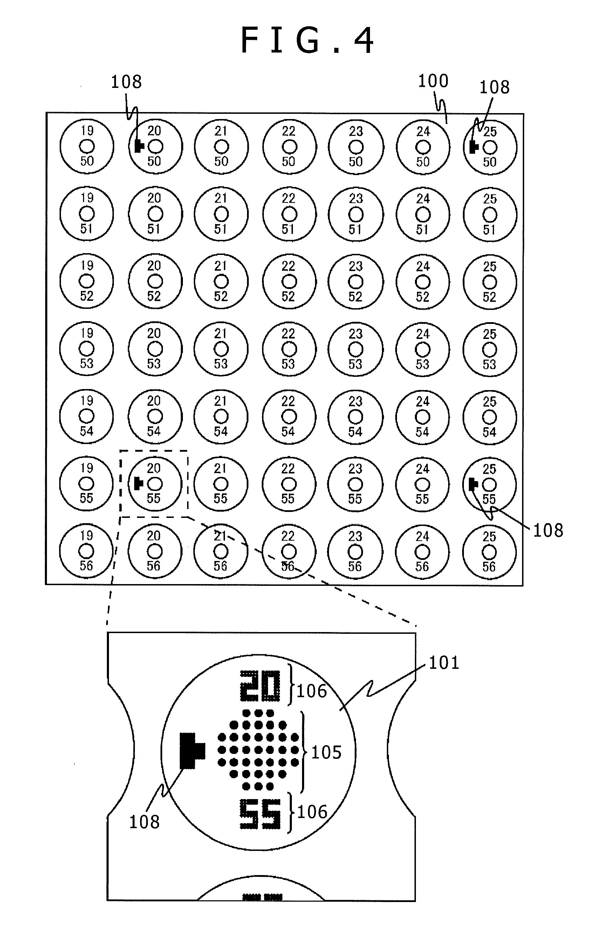

Fig. 4 is a view showing a culturing sheet and a hole structure in the culturing sheet according to a third embodiment.
Fig. 5A is a view for explaining an example of a manufacturing method of the culturing sheet according to the first embodiment.
Fig. 5B is a view for explaining the example of the manufacturing method of the culturing sheet according to the first embodiment.
Fig. 5C is a view for explaining the example of the manufacturing method of the culturing sheet according to the first embodiment.
Fig. 6 is a diagram for explaining an effect of the culturing sheet according to the first embodiment.

### Description of Embodiments

A detailed explanation will be given of various embodiments for culturing cells by using a culturing sheet, and realizing formation thereof in a three-dimensional tissue which is a cell aggregate, or a two-dimensional plane tissue in reference to the drawings as follows. Incidentally, in the present specification, a sheet having a structure of partitioning a culturing region and formed with plural protrusions at an inner portion of the partitioning structure is referred to as a culturing sheet in contrast to a conventional nanopillar sheet.

### First Embodiment

First embodiment shows an example of applying a culturing sheet to a culturing equipment material of a multiwells plate having 24 holes which is a culturing sheet holding member. The culturing sheet is formed by a substance which does not effect an advance influence on a cell. The culturing sheet according to the present embodiment uses polystyrene. However, the material is not naturally limited to polystyrene. Although according to the present embodiment, the multiwells plate having 24 holes is used as the holding member, other multiwells plate having different number of holes, or a chamber slide or the like will do.

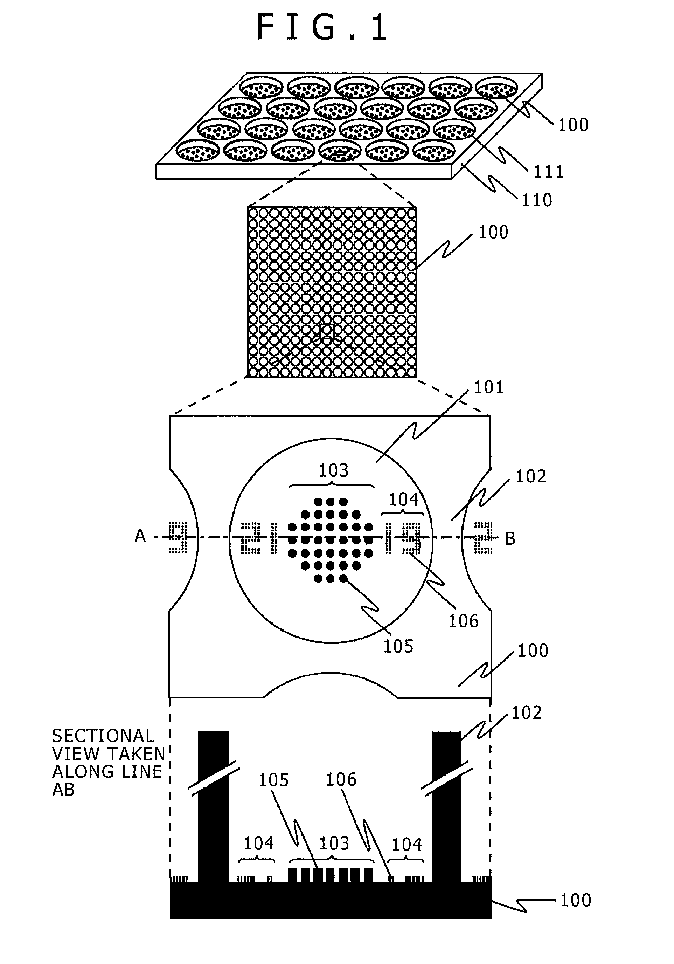

In a culturing vessel 110 of the multiwells plate of 24 holes in Fig. 1, a culturing sheet 100 is bonded to a bottom face of a cylindrical hole having a diameter of about 16 mm that is referred to as well 111 by a method of ultrasonic welding or the like. Plural pieces of holes per one sheet are present at the culturing sheet 100, and the holes 101 are individually isolated from each other by a partitioning structure 102. The hole 101 is a minimum unit of a culturing region, and is configured by a local culturing region 103 locally arranged with protrusions, that is, pillars at inside of the hole and an identification mark region 104.

The local culturing region 103 which is disposed at the bottom face of the hole 101 is configured by plural pillars. Incidentally, a pillar which is disposed at the local culturing region 103 at inside of the hole is defined as a local culturing region pillar 105, and a pillar of the identification mark region 104 is defined as an identification mark pillar 106.

According to the present embodiment, there is used the culturing sheet 100 in which a diameter of each hole 101 partitioned by the partitioning wall 102 which is the partitioning structure above the culturing sheet 100 is made to be 200 µm, a pitch between the holes is made to be 220 µm, a diameter of the local culturing region 103 is made to be 80 µm, a height, a pillar diameter, and a pillar pitch of the local culturing region pillar 105 are respectively made to be 1 µm, 2µm, and 4 µm, and a height, a pillar diameter, and a pillar pitch of the identification mark pillar 106 are respectively made to be 0.1 µm, 0.5 µm, and 1 µm.

In the culturing sheet 100, the partitioning wall 102, and the hole 101 isolated thereby, the local culturing region 103 and the identification mark region 104 which are formed at the inner portion of the hole 101 are integrally formed by the same material.

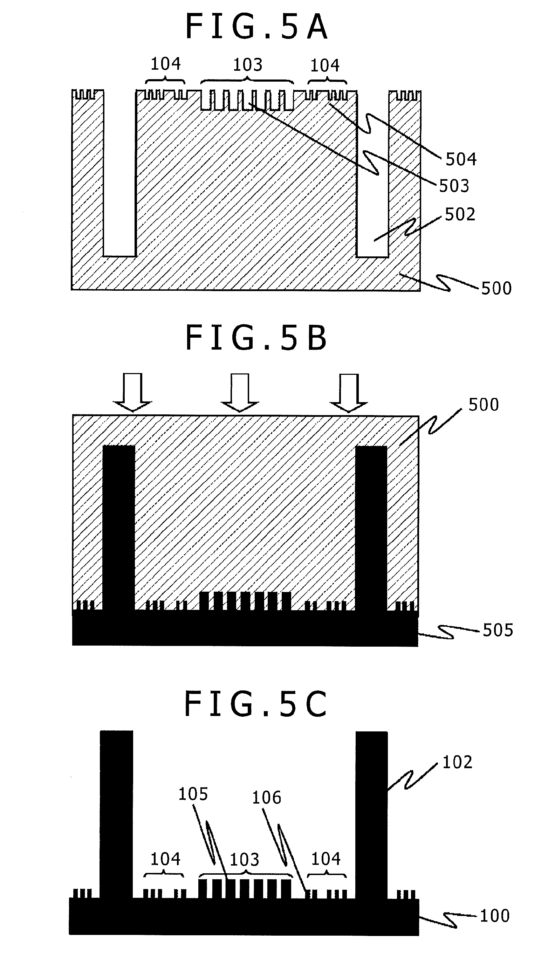

An example of a manufacturing method of the culturing sheet according to the embodiment will be explained in reference to Fig. 5A, Fig. 5B, and Fig. 5C. First, as shown in Fig. 5A, holes 502, 503, and 504 respectively in correspondence with the partitioning wall 102, the local culturing region 103 configured by the local culturing holes, and the identification mark region 104 configured by the identification mark holes are formed at the same silicon substrate 500. As shown in Fig. 5B, a pattern is transcribed by a so-called thermal imprinting method in which a polystyrene sheet 505 is pressed to the silicon substrate 500 while heating the polystyrene sheet 505. Thereby, the cell culturing sheet 100 shown in Fig. 5C is formed.

The cell culturing sheet according to the present embodiment can be formed in one motion by previously forming the partitioning wall hole 502, the local culturing hole 503, and the identification mark hole 504 which configure an inverted pattern of the cell culturing sheet at the silicon substrate which becomes a die. Incidentally, the forming method is an example, and a pattern can naturally be transcribed in one motion similarly also by using electrocast nickel or quartz instead of silicon as a die material.

As shown in Fig. 1, according to the present embodiment, the identification mark region 104 is configured by two digits numerals on left and right sides of the local culturing region 103. The numerals on the left side indicate a column of a hole arrangement, the numerals on the right side indicate a row, and a position of the hole can be read by the numerals. Although according to the present embodiment, all of the holes are serially attached with two digit numerals, it is not necessarily needed that the numerals are configured by two digits, but the numerals may be attached at intervals of plural pieces of holes. Not numerals but characters, for example, alphabet, or signs may naturally be used.

A spheroid which is a three-dimensional tissue having a uniform grain size can be held at a center portion of the culturing region and can be held at a target position by the local culturing region 103 as disclosed in Patent Literature 2.

Incidentally, although present embodiment shows an example of aligning the local culturing region pillars at a vicinity of a center in the culturing region, it is not necessarily needed that the local culturing region pillars include a center, but the pillars may naturally be aligned at a desired region in the culturing region. Although examples of forming a circular pillar region are shown as the local culturing region, the pillar region may naturally be formed by a quadrangular shape or a polygonal shape.

Although the identification mark region 104 is arranged within the same region of the hole 101 at a position different from that of the local culturing region 103, it is necessary to prevent a spheroid from being adhered to the identification mark region. This is for preventing a situation where a spheroid of a desired size cannot be obtained by forming two spheroids at the local cell culturing region 103 and the identification mark region 104, or the identification mark cannot optically be recognized by adhering a spheroid to the identification mark region 104 to shield the identification mark.

In contrast thereto, according to the identification mark region pillar 106 of the identification mark region 104 of the present embodiment, a height thereof is made to be lower than that of the cell culturing region pillar, or/and a diameter, or/and a pitch thereof is made to be smaller than that (those) of the cell culturing region pillar. Thereby, a cell can be prevented from being adhered to the identification mark region.

It has been confirmed by an experiment of the inventors that in, for example, hepatic cell, an optimal pillar diameter is 2 µm (pillar pitch 4 µm), and an optimal pillar height is 1 µm, and an adhesion of the cell is weakened only by halving even one of the values. Therefore, the cell can be prevented from being adhered to the identification mark region by enlarging the differences between the cell culturing region pillar 105 and the identification mark region pillar 106.

The adsorption of the cell can be prevented by lowering the height of the identification mark region pillar 106. However, it is necessary that a person can optically recognize the pillar by an optical method, for example, an optical microscope which is one of objects of the present invention, and there is a lower limit value of the height of the pillar.

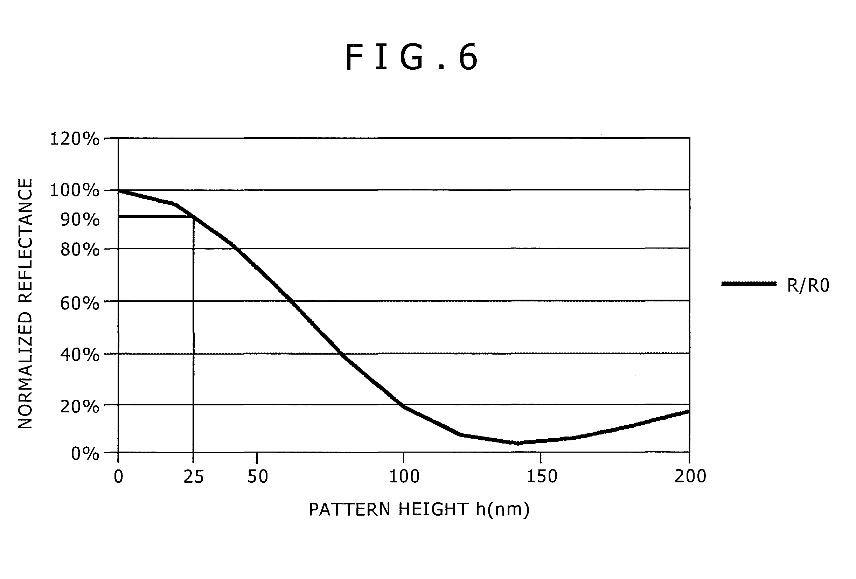

Fig. 6 shows a result of calculating a reflectance when the pitch is 100 nm and a pattern width is 500 nm by using polystyrene and a wavelength of 550 nm by a simulation. In Fig. 6, reflection intensities at respective heights are designated by notation R, and normalization is carried out by a reflection intensity of R₀ when the pattern height is 0 nm, that is, when there is not a pattern. As shown in Fig. 6, the reflection intensity is lowered up to the pattern height h of 140 nm, that is, the pattern is darkened compared with surroundings. When there is a difference of 10 % therebetween, the pattern can be read by a machine. Therefore, a character can sufficiently be read when an intensity ratio is equal to or less than 90 %, that is, when the pattern height is equal to or more than 25 nm.

The identification mark region can be reduced by reducing the diameter and the pitch. Thereby, an area of the identification mark region which is not directly related to the cell culturing can be reduced. Or, an optical recognizability by an optical microscope or the like can be improved by reducing a pillar diameter or a pillar pitch.

Although according to the present embodiment, the optical microscope is used as an inspection method, the inspection which is carried out by an optical or an electrooptical method such as a laser microscope, an electron microscope or the like may naturally be used.

Although the height of the identification mark pillar is the same as or lower than the cell culturing region pillar according to the present embodiment, even when the heights of the cell culturing region pillar and the identification mark pillar differ from each other, the difference is about several µm at most.

Here, an explanation will be given of a relationship between a region or a field of view which can be observed in one motion, and a depth of focus or a focal depth which can be observed at the same focusing. In a case of using a light source wavelength (λ) of 0.55 µm, a number of field of view (F) of 26, and a magnification (m) of × 10 of an eye lens, and using a magnification (M) of an object lens of × 10, and a lens of a numerical aperture (NA) of 0.3, an actual field of view which can be observed in one motion becomes F/M and is 2.6 mm. About 12 pieces of holes can be observed in one motion diagonally since the pitch of the hole is 0.22 mm. The focal depth at this occasion can be represented by λ/(2 ×(NA)²) and therefore, is about ±3 µm. In a case of using a light source wavelength (λ) of 0.55 µm, a number of field of view (F) of 26, and a magnification (m) of × 10 of an eye lens, and using a magnification (M) of an object lens of × 20, and a numerical aperture (NA) of 0.46, an actual field of view is 1.3 mm, and a focal depth is ±1.3 µm. Although the focal depth is changed by a lens performance in this way, the focal depth is not changed in digit so far as the field of view is not considerably changed. A range which is actually used for observation in one motion is one piece through about ten and several pieces in one field of view. Therefore, an observed focal depth is sub µm to several tens µm or less. Therefore, a spheroid can be observed and a mark can be identified by only moving a focusing position for observing a spheroid and a focusing position of the identification mark simultaneously or by small amounts.

A culturing equipment material which is high in a culturing efficiency and is easy to be used can be realized by forming a culturing sheet including a structure of the present embodiment in this way.

### Second Embodiment

Second embodiment shows an example of applying a culturing sheet to a culturing equipment material of a chamber plate which is a culturing sheet holding member, and shows a case where an identification mark of a culturing sheet is fabricated not by a pillar but by a line.

According to the present embodiment, a diameter of each hole 101 partitioned by the partitioning wall 102 which is the partitioning structure above the culturing sheet 100 is made to be 130 µm, a pitch of the hole is made to be 150 µm, a diameter of the local culturing region 103 is made to be 60 µm, and a height, a pillar diameter, and a pillar pitch of the local culturing region pillar 104 are respectively made to be 2 µm, 1 µm, and 2.5 µm. The culturing sheet 100 where a height and a pattern width of an identification mark line 108 are respectively 0.25 µm and 5 µm is used.

As a result, adhesion of a cell can be restrained by making the height of the identification mark lower than the height of the pillar similar to the first embodiment. An adhesive property of a cell can further be restrained by configuring the identification mark not by an aggregate of fine patterns such as pillars but by the line as in the present embodiment.

### Third Embodiment

Third embodiment shows a cell culturing sheet having numbers of rows and columns indicating positions of the holes, and alignment marks 108 which are arranged at intervals of 5 pieces of the rows and the columns at the identification mark regions 104 in the individual holes 101. Although in this case, the alignment marks 108 are arranged at intervals of 5 pieces of the rows and the columns, the alignment marks 108 may be attached at intervals of several hundreds pieces of the rows and the columns, or may be attached to all of the respective holes. It is preferable for the alignment mark to reduce any or all of values of a pillar diameter(s), a pillar pitch(s), and a pillar height(s) more than those of the local culturing region pillars similar to the identification mark pillars.

A position alignment of a defect inspection device, or a microscope for observation can be carried out by the pillar of the alignment mark 108. The alignment can be adjusted more easily and more swiftly by making a shape of the alignment mark 108 easy to be read by an automatic alignment device of the defect inspection device or the like, registering an image thereof to the device, and subjecting the image to a comparison inspection.

According to the various embodiments explained above, formation of a three-dimensional tissue can be realized under an environment with less stress while maintaining an activity by urging a cell movement which is a function inherent to the cell by using only the single material, the identification mark can be read without being shielded by the three-dimensional tissue, and therefore, the three-dimensional tissue and a two-dimensional plane tissue can be evaluated and managed.

The identification mark can be read by restraining trapping of the cell by the identification mark by making the height of the identification mark lower than the height of the pillar which cultivates a cell.

Or, optical refraction of the pillar is made to differ by configuring the identification mark by a shape of the pillar a pillar diameter of which is smaller than a diameter of the pillar which cultivates cells, or/and a pillar height of which is lower than that of the pillar which cultivates cells, a contrast in optical observation is improved, and the identification mark can further be made easy to read.

Or, trapping of the cell by the identification mark can be restrained while improving an optical recognizability in optical observation or the like by making the height of the identification mark equal to or higher than 0.025 µm and equal to or lower than 0.5 µm.

Or, an automatic alignment or automatic focus adjustment can be facilitated by fabricating a shape easy to read by an automatic reading device in each hole or a specific hole as the identification mark.

A person is easy to recognize coordinates more directly by configuring the identification mark by the numeral or the character, for example, alphabet, or a sign.

Incidentally, the present invention is not limited to the embodiments described but includes various modification examples. For example, the embodiments described above have been explained in details for explaining to be easy to understand the present invention, and are not necessarily limited to what includes all of configurations of the explanation. A portion of the configuration of a certain embodiment can be substituted for by a configuration of other embodiment, and a configuration of other embodiment can be added to a configuration of a certain embodiment. Addition, deletion, or substitution of other configuration can be carried out for portions of configurations of the respective embodiments.

### List of Reference Signs

100 cell culturing sheet, 101 hole, 102 partitioning wall, 103 local culturing region, 104 identification mark region, 105 local culturing region pillar, 106 identification mark pillar, 107 identification mark line, 108 alignment mark, 110 culturing vessel, 111 well, 500 silicon substrate, 502 partitioning wall hole, 503 local culturing hole, 504 identification mark hole, 505 polystyrene sheet

## Claims

1. A culturing sheet for culturing a cell, comprising:
a partitioning wall;
a hole isolated by the partitioning wall;
a culturing region formed with a plurality of protrusions a height of which is lower than a height of the partitioning wall at a portion of a bottom face of the hole; and
an identification mark formed at a position different from a position of the culturing region at the bottom face of the hole.

2. The culturing sheet according to claim 1, wherein the identification mark is formed by a plurality of protrusions.

3. The culturing sheet according to claim 2, wherein a diameter of the protrusion of the identification mark is smaller than a diameter of the protrusion of the culturing region.

4. The culturing sheet according to claim 2, wherein a height of the protrusion of the identification mark is lower than the height of the protrusion of the culturing region.

5. The culturing sheet according to claim 4, wherein the height of the protrusion of the identification mark falls in a range of 0.025 µm through 0.5 µm.

6. The culturing sheet according to claim 1, wherein the identification mark represents a position of the hole at the culturing sheet.

7. The culturing sheet according to claim 6, wherein the identification mark represents the position of the hole by forming a numeral or a character.

8. A culturing equipment material using the culturing sheet according to claim 1, comprising:
a culturing vessel formed with a plurality of wells,
wherein the culturing sheet is held at a bottom portion of the culturing vessel.

9. The culturing equipment material according to claim 8, wherein the identification mark is formed by a plurality of protrusions.

10. The culturing equipment material according to claim 9, wherein a diameter of the protrusion of the identification mark is smaller than a diameter of the protrusion of the culturing region.

11. The culturing equipment material according to claim 10, wherein a height of the protrusion of the identification mark is lower than a height of the protrusion of the culturing region.

12. The culturing equipment material according to claim 11, wherein the height of the protrusion of the identification mark falls in a range of 0.025 µm through 0.5 µm.

13. A manufacturing method of a culturing sheet for culturing a cell, comprising:
forming a die substrate of a culturing sheet including a partitioning wall, a hole isolated by the partitioning wall, a culturing region formed with a plurality of protrusions having a height lower than a height of the partitioning wall at a portion of a bottom face of the hole, and an identification mark region formed at a position different from a position of the culturing region at the bottom face of the hole, and formed with a plurality of protrusions a height of which is lower than the height of the protrusion of the culturing region; and
pressing a material of the culturing sheet to the die substrate while heating the material.

14. The manufacturing method of a culturing sheet according to claim 13, wherein a silicon substrate, electrocast nickel or quartz is used as the die substrate.

15. The manufacturing method of a culturing sheet according to claim 13, wherein a polystyrene sheet is used as the material of the culturing sheet.
